# EUROPÄISCHE PATENTSCHRIFT

(11) **EP 0 765 683 B1**
(45) Veröffentlichungstag und Bekanntmachung des Hinweises auf die Patenterteilung: **01.07.1998**
(21) Anmeldenummer: 96112934.3
(22) Anmeldetag: 12.08.1996
(51) Int. Cl.: B01D 63/04, A61M 1/18

(54) **Vorrichtung zur Behandlung von Flüssigkeiten, insbesondere von Blut**
Device for treating liquids, in particular blood
Appareil de traitement de liquides, en particulier du sang

(30) Priorität: 25.09.1995 DE 19535346
(43) Veröffentlichungstag der Anmeldung: 02.04.1997
(73) Patentinhaber: MEDOS Medizintechnik GmbH, 52222 Stolberg (DE)
(72) Erfinder: Eilers, Rolf, Dipl.-Ing., 52066 Aachen (DE); Hildinger, Karl-Heinz, 52080 Aachen (DE); Mückter, Helmut, Dr.-med. Dipl.-Ing., 52076 Aachen (DE)
(74) Vertreter: Castell, Klaus, Dr.-Ing.

(56) Entgegenhaltungen:
- EP-A- 0 187 708
- EP-A- 0 471 921
- EP-A- 0 507 722
- WO-A-91/04758
- DE-A- 2 514 763
- US-A- 4 289 623
- US-A- 4 929 259
- PATENT ABSTRACTS OF JAPAN vol. 013, no. 423 (C-638), 20.September 1989 & JP-A-01 160564 (UBE IND LTD), 23.Juni 1989,
- PATENT ABSTRACTS OF JAPAN vol. 014, no. 232 (C-0719), 17.Mai 1990 & JP-A-02 059016 (UBE IND LTD), 28.Februar 1990,
- PATENT ABSTRACTS OF JAPAN vol. 013, no. 151 (C-584), 12.April 1989 & JP-A-63 310605 (TOYOBO CO LTD), 19.Dezember 1988,
- PATENT ABSTRACTS OF JAPAN vol. 013, no. 115 (C-578), 20.März 1989 & JP-A-63 290574 (UBE IND LTD), 28.November 1988,

## Beschreibung

Die Erfindung betrifft eine Vorrichtung zur Behandlung von Flüssigkeiten, insbesondere von Blut, mit mehreren Kammern, die von ineinander angeordneten Rohren gebildet sind und einen Einlauf und einem Auslauf aufweisen und in denen Hohlkammermembranen angeordnet sind, wobei die Kammern so miteinander verbunden sind und Einlauf und Auslauf so angeordnet sind, daß in den Kammern eine Strömung in Längsrichtung der Rohre bewirkt wird.

Eine gattungsgemäße Vorrichtung ist aus der JP-A-1 160 564 bekannt, wenn das Netz als Rohr mit einer offenen Struktur angesehen wird. Auch die DE-A-25 14 763, die JP-A-2 059 016 und die JP-A-63 310 605 beschreiben ähnliche Vorrichtungen.

Diese Vorrichtungen eignen sich jedoch nicht für mehrere Funktionen, wie z. B. zum Temperieren und Oxidieren von Blut.

Bei der aus der US-A-5 270 004 bekannten Vorrichtung fließt das Blut durch einen zentral am Boden angeordneten Bluteinlauf in die Vorrichtung und durch Löcher in einer porösen Rohrwand radial in eine diese Rohrwand umgebende Ringkammer, in der von einer Heizflüssigkeit durchflossene Hohlfasermembran angeordnet sind. Daraufhin fließt das Blut weiter durch Öffnungen in einem zweiten Rohr in einen weiteren ringförmigen Hohlraum, in dem von einem Gas durchflossene Hohlfasermembranen angeordnet sind. Dieser Raum ist von einer dritten flüssigkeitdurchlässigen Ringwandung umgeben, durch die das Blut radial in einen ringförmigen Sammelkanal und von dort zum Blutauslauf fließt.

Diese Vorrichtung eignet sich zum Temperieren und Oxigenieren von Blut und sie zeichnet sich durch einen kompakten Aufbau aus. Schon dem Aufbau des Gerätes ist jedoch zu entnehmen, daß die Hauptströmungsrichtung des Blutes diagonal vom zentralen Einlaß an der Unterseite zum Auslaß im Umfangsbereich an der Oberseite der Vorrichtung verläuft und in den Bereichen oben mittig und unten im Außenbereich mit Totzonen zu rechnen ist. In der Praxis werden sich daher unterschiedliche Strömungsgeschwindigkeiten ergeben, die von einer maximalen Strömungsgeschwindigkeit im Bereich der beschriebenen Diagonale zu den Totzonen hin abnimmt. Außerdem entstehen leicht Durchbrüche in Folge von Kanalbildung durch Fertigungstoleranzen. Diese unterschiedlichen Strömungsgeschwindigkeiten führen zu lokal unterschiedlichem Stoff- und Wärmeaustausch zwischen den Hohlkammermembranen und dem Blut und somit zu einer verminderten Austauschleistung.

Der Erfindung liegt daher die Aufgabe zugrunde, eine gattungsgemäße Vorrichtung vorzuschlagen, die eine möglichst gleichmäßige Durchströmung und verschiedene Funktionen, wie zum Beispiel das Temperieren und Oxidieren von Blut ermöglicht.

Diese Aufgabe wird mit einer Vorrichtung mit den Merkmalen des Patentanspruchs 1 gelöst.

Mit der erfindungsgemäßen Vorrichtung wird eine axiale Strömungsrichtung erzwungen. Dies wird dadurch erreicht, daß die Kammern jeweils nur an ihren Enden einen Zu- oder Ablauf haben. Dadurch wird der gesamte in den Kammern geführte Fluidstrom gezwungen die einzelnen Kammern in Längsrichtung zu durchströmen, bevor er aus der Vorrichtung austreten kann. Dabei kann das Blut entweder in den Kammern oder in den Hohlkammermembranen geführt werden.

Eine besonders kompakte Bauweise der Vorrichtung ist zu erzielen, wenn das Rohr der Außenwandung einer inneren Kammer die Innenwandung einer äußeren Kammer bildet. Die einzelnen Kammern liegen dadurch direkt aneinander, wodurch Material eingespart und das Bauvolumen verringert wird. Außerdem bewirkt die kompakte Bauweise eine Verkürzung der Verbindungskanäle zwischen den einzelnen Kammern.

Ein vereinfachter Aufbau und eine Verkürzung der Verbindungskanäle wird auch dadurch erreicht, daß die Kammern im wesentlichen gleichlang sind und die axialen Enden der Hohlkammermembranen auf einer Ebene liegen. Vorzugsweise sind auch die in den Kammern angeordneten Hohlkammermembranen im wesentlichen gleichlang. Dies vereinfacht den Aufbau der Vorrichtung und erlaubt eine kostengünstige Herstellung, da das Vergießen und Schneiden der in den Kammern angeordneten Hohlkammermembranen jeweils in einem Arbeitsgang erfolgen kann.

Die kompakte Bauweise hat nicht nur herstellungstechnische Vorteile, sondern führt auch zu einer Verringerung des Priming-Volumens, d. h. des Luftvolumens, das bei einer Erstbefüllung der Vorrichtung durch das eingeleitete Fluid verdrängt werden muß.

Vorteilhaft ist es, wenn die Rohre leicht kegelförmig ausgebildet sind. Dies führt zu einer kostengünstigeren gießtechnischen Herstellung. Außerdem werden die Reibungskräfte bei der Montage der Einzelelemente der Vorrichtung verringert und somit die mechanische Belastung der Fasern während der Montage verringert.

Um die Schubspannungen im Einlaufbereich zu minimieren weist bei einer vorteilhaften Ausgestaltungsform der Vorrichtung der Einlauf einen Zyklon auf. Der im Einlauf vorgesehene Zyklon erlaubt es darüberhinaus, schon im Einlaufbereich Luftbläschen aus dem Blut zu entfernen.

Eine platzsparende Integration des Zyklons in die Vorrichtung wird dadurch erzielt, daß der Zyklon zumindest teilweise in einem Rohr angeordnet ist. Dies führt zu kurzen Strömungswegen des Blutes und einem kompakten Aufbau der Vorrichtung.

Vorteilhaft ist es, wenn der Auslauf als Ringkanal mit in Strömungsrichtung zunehmendem Querschnitt ausgebildet ist. Dies erlaubt bei minimalem Priming-Volumen eine schonende Abführung des Blutes aus der zuletzt durchströmten Kammer in den Auslauf.

In den einzelnen Kammern sind verschiedene Arten von Hohlkammermembranen angeordnet. Prinzipiell ist es möglich auch in einer Kammer verschiedene Arten von Hohlkammermembranen unterzubringen. Durch eine geeignete Ausbildung der Anschlüsse können diese auch von verschiedenen Medien durchflossen werden. Die Hohlkammermembranen können aus Membranplatten gebildet sein oder vorzugsweise aus Hohlfasern.

Ein einfacher Aufbau der Vorrichtung sieht jedoch vor, eine Kammer mit flüssigkeitsdurchströmbaren Hohlkammermembranen zu versehen, um das Blut zu temperieren und eine weitere Kammer mit gasdurchströmbaren Hohlkammermembranen, um das Blut zu oxygenieren. Vorteilhaft ist es dabei das Blut zunäçhst zu temperieren und anschließend zu oxygenieren, damit die Oxygenierung bei einheitlichen Temperaturverhältnissen durchgeführt werden kann. Da die Gaslöslichkeit im Blut und anderen Flüssigkeiten von der Temperatur abhängt, ist es wichtig, daß das Blut mit der Temperatur oxygeniert wird, mit der es in den Körper eintritt. Wenn das Blut nach der Oxygenierung erwärmt wird, besteht die Gefahr, daß der Sättigungsgrad überschritten wird und durch freie Luftbläschen im Körper Embolien entstehen.

Da üblicherweise eine größere Austauschfläche zwischen Membran und Blut am Oxygenator notwendig ist als am Wärmetauscher, ist es vorteilhaft den Oxygenator radial außerhalb des Wärmetauschers anzuordnen. Zur Erzielung einer schnellen Oxygenierung des die Vorrichtung durchströmenden Blutes wird vorgeschlagen, in einer Kammer poröse Hohlfasermembranen anzuordnen. Je nach Einsatzbereich der Vorrichtung können jedoch als Hohlfasermembranen unterschiedlichste Membranmaterialien verwendet werden und es steht frei, entweder die zu behandelnde Flüssigkeit durch die Membranen und die Behandlungsfluide durch die Kammern oder die zu behandelnde Flüssigkeit durch die Kammern und die Behandlungsfluide, d.h. Flüssigkeiten oder Gase, durch die Hohlkammermembranen zu leiten. Außerdem können die Kammern je nach Einsatzbereich parallel oder hintereinander durchströmt werden.

Eine vorteilhafte Verwendung der beschriebenen Vorrichtung sieht vor, daß durch die Kammern Blut und durch einen Teil der Hohlkammermembranen eine Temperierflüssigkeit und durch einen anderen Teil der Hohlkammermembranen Gas geleitet wird. Die Führung des Blutes durch die Kammern erlaubt bei der erfindungsgemäßen Vorrichtung eine Minimierung des Priming-Volumens und einen schonenden Bluttransport. Die Beaufschlagung der Hohlkammermembranen durch unterschiedliche Medien erlaubt währenddessen eine definierte Konditionierung der durch die Vorrichtung hindurchgeführten Flüssigkeit.

Ein Ausführungbeispiel der erfindungsgemäßen Vorrichtung ist in der Zeichnung dargestellt und wird im folgenden näher beschrieben.

Es zeigt:
- Figur 1: einen axialen Schnitt durch eine erfindungsgemäße Vorrichtung,
- Figur 2: eine Draufsicht auf die Vorrichtung nach Figur 1,
- Figur 3: einen Schnitt durch die Vorrichtung in Figur 1 längs der Linie III III und
- Figur 4: eine Ansicht der Vorrichtung von unten.

Die Vorrichtung 1 dient dem Temperieren und Oxygenieren von Blut und wird in der Fachsprache als Oxygenator-Wärmetauscher bezeichnet. Sie besteht im wesentlichen aus drei koaxial angeordneten Rohren 2, 3, 4, die zwischeneinander eine innere Kammer 5 und eine äußere Kammer 6 begrenzen. Die Enden der inneren Kammer 5 und der äußeren Kammer 6 sind mit einer Vergußmasse 7 verschlossen, die in den Kammern 5, 6 angeordnete Hohlfasermembranen so hält, daß die Öffnungen der Hohlfasermembranen außerhalb der Kammern liegen.

Die Rohre 2, 3 und 4 sind leicht konisch ausgebildet, so daß ihr Querschnitt an der Oberseite 10 der Vorrichtung 1 größer ist, als an der Unterseite 11.

Das innerste Rohr 2 umschließt einen Hohlraum 12 an dessen Oberseite ein Zyklon 13 angeordnet ist. Dieser Zyklon 13 hat einen Eingang, der den Bluteinlauf 14 bildet und einen Ausgang 15, der am oberen Ende des innersten Rohres 2 in die innere Kammer 5 führt.

Im unteren Bereich der Kammern 5 und 6 sind im mittleren Rohr 3 Öffnungen 16 vorgesehen, die die innere Kammer 5 mit der äußeren Kammer 6 verbindet.

Das äußerste Rohr 4 erweitert sich radial am oberen Ende in einem Umfangsbereich 17, um spiralartig in einen Blutauslauf 18 zu münden.

Die Oberseite 10 ist mit einem oberen Deckel 19 und die untere Seite 11 der Vorrichtung 1 ist mit einem unteren Deckel 20 verschlossen.

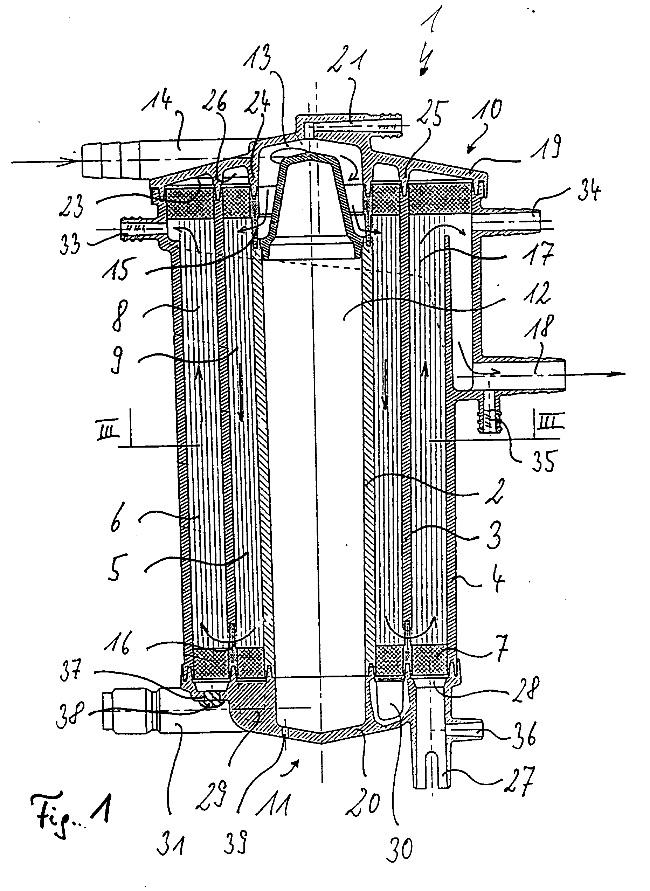

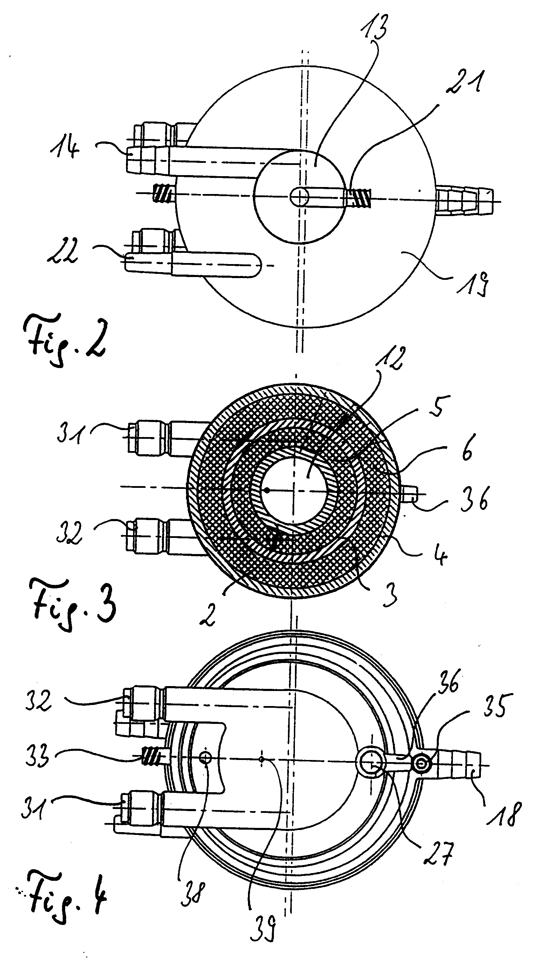

Im oberen Deckel 19 ist der Bluteinlauf 14 so angeordnet, daß der Zyklon 13 tangential angeströmt wird. Außerdem ist am obersten Punkt des Zyklons 13 eine Entlüftung 21 vorgesehen, mit der im Zyklon 13 anfallende Luft entfernt werden kann. Darüberhinaus ist im oberen Deckel 19 ein Gaseinlauf 22 (vergleiche Figur 2) angeordnet, der in einen vom Deckel 19 und der Vergußmasse 7 begrenzten oberen Gasraum 23 führt. Sich vom Deckel 19 axial zu den Rohren 2 und 3 erstreckende Stege 24, 25 begrenzen einen oberen Flüssigkeitsraum 26, der der Umlenkung von in den Hohlfasermembranen 9 der inneren Kammer 5 geführtem Wasser dient.

Der untere Deckel 20 weist einen Gasauslauf 27 auf, der als axial geführtes Rohr in einen zwischen unterem Deckel 20 und Vergußmasse 7 unterhalb der äußeren Kammer 6 gebildeten unteren Gasraum 28 mündet. Außerdem weist der untere Deckel einen durch zwei Platten 29 (die andere Platte ist in der Darstellung weggebrochen) unterbrochenen Ringkanal 30 auf, in dessen eine Seite ein Wassereinlauf 31 und in dessen andere Seite ein Wasserauslauf 32 mündet.

Darüberhinaus weist die Vorrichtung 1 am oberen Ende der äußeren Kammer 6 gegenüber dem Blutauslaß 18 eine Temperaturmeßöffnung 33 auf, in der eine Temperaturmeßsonde angeordnet werden kann und die auch als Entlüftung einsetzbar ist. Ebenfalls am oberen Ende der äußeren Kammer 6, aber direkt oberhalb des Blutauslasses 18 ist ein weiterer Blutauslaß 34 für eine Rezikulationslinie vorgesehen, durch den ein Blutvolumenstrom im Bypaß in ein Reservoir (nicht gezeigt) geleitet werden kann. An der Unterseite des Blutauslaufes 18 ist eine Probeöffnung 35 vorgesehen, an der Blutproben entnehmbar oder Meßgeräte einführbar sind. Ein waagerechter Anschlußstutzen 36 ist am Gasauslaß 27 vorgesehen, um gasseitig Druck oder Temperatur zu messen. Gegenüber dem Gasauslaß 27 ist im Gasraum 28 eine Drucköffnung 37 vorgesehen, die durch einen Stöpsel 38 verschlossen ist. Diese Drucköffnung 37 ist so ausgebildet, daß sie sich bei einem überhöhten Druck auf der Gasseite öffnet. Eine Ausgleichsöffnung 39 am unteren Ende des Hohlraums 12 im Deckel 20 dient dem Druckausgleich in diesem Hohlraum 12.

Beim Betreiben der Vorrichtung 1 wird ein Warmwasserstrom durch den Wassereinlauf 31 auf die eine Seite des Ringkanals 30 und von dort in die eine Hälfte der in der inneren Kammer 5 angeordneten Hohlfasermembranen 9 geleitet. In den Hohlfasermembranen 9 steigt das Wasser bis in den Flüssigkeitsraum 26 im oberen Deckel 19, verteilt sich dort und strömt durch die restlichen Hohlfasermembranen 9 in der inneren Kammer 5 zurück nach unten auf die andere Seite des Ringkanals 30 und verläßt von dort die Vorrichtung über den Wasserauslaufs 32.

Über den Gaseinlauf 22 strömt Gas in den oberen Gasraum 23 im oberen Deckel 19 und von dort durch die Hohlfasermembranen 8 in der äußeren Kammer 6 zum unteren Gasraum 28 im unteren Deckel 20 der Vorrichtung 1 und verläßt durch den Gasauslauf 27 die Vorrichtung.

Blut fließt über den Bluteinlauf 14 in den Zyklon 13, strömt im Zyklon 13 spiralartig nach unten und gelangt durch den Ausgang 15 in die innere Kammer 5. Dort strömt das Blut an den Hohlfasermembranen 9 entlang und wird entsprechend der Temperatur des in den Hohlfasermembranen geführten Wassers temperiert. Am unteren Ende der Kammer 5 sind Öffnungen 16 vorgesehen, durch die das Blut in die äußere Kammer 6 strömt, um dort entlang den gasdurchströmten Hohlfasermembranen 8 nach oben zu strömen. Dabei wird das Blut mit Sauerstoff angereichert. Am oberen Ende der äußeren Kammer 6 angelangt, strömt das Blut auf einer Kreislinie zum Blutauslauf 18, wo es die Vorrichtung 1 verläßt.

Die innere Kammer 5 und die äußere Kammer 6 sind jeweils an ihrem obersten Ende mit Öffnungen versehen, damit in den Kammern aufsteigende Luft durch diese Öffnungen aus den Kammern ausdringen kann. Luft aus der inneren Kammer 5 strömt durch den Ausgang 15 in den Zyklon 13 und von dort durch die Entlüftung 21 aus der Vorrichtung und Luft in der äußeren Kammer 6 gelangt durch die Temperaturmeßöffnung 33 oder den weiteren Blutauslaß 34 aus der Vorrichtung. Dadurch ist sowohl bei der Erstbefüllung der Vorrichtung als auch während des Betriebes eine optimale Entlüftung gewährleistet.

## Patentansprüche

1. Vorrichtung zur Behandlung von Flüssigkeiten, insbesondere von Blut, mit mehreren Kammern (5, 6), die von ineinander angeordneten Rohren (2, 3, 4) gebildet sind und einen Einlauf (14) und einen Auslauf (18) aufweisen und in denen Hohlkammermembranen (8, 9) angeordnet sind, wobei die Kammern (5, 6) so miteinander verbunden sind und Einlauf (14) und Auslauf (18) so angeordnet sind, daß in den Kammern (5, 6) eine Strömung in Längsrichtung der Rohre (2, 3, 4) bewirkt wird,
***gekennzeichnet durch***
- in den einzelnen Kammern (5, 6) angeordnete verschiedene Arten von Hohlkammermembranen (8, 9),
- einen oberen und einen unteren Deckel (19, 20) mit Anschlüssen (22, 27, 31, 32) und
- sich vom oberen Deckel (19) axial zu den Rohren (2, 3) erstreckende Stege, so daß die Hohlkammermembranen (8, 9) von unterschiedlichen Medien durchflossen werden.

2. Vorrichtung nach Anspruch 1, ***dadurch gekennzeichnet, daß*** das Rohr (3) der Außenwandung einer inneren Kammer (5) die Innenwandung einer äußeren Kammer (6) bildet.

3. Vorrichtung nach einem der vorhergehenden Ansprüche**, *dadurch gekennzeichnet, daß*** die Kammern (5, 6) im wesentlichen gleich lang sind und die axialen Enden der Hohlkammermembranen auf einer Ebene liegen.

4. Vorrichtung nach einem der vorhergehenden Ansprüche, ***dadurch gekennzeichnet, daß*** die Hohlkammermembranen (8, 9) im wesentlichen gleichlang sind.

5. Vorrichtung nach einem der vorhergehenden Ansprüche, ***dadurch gekennzeichnet, daß*** die Rohre (2, 3, 4) leicht kegelförmig ausgebildet sind.

6. Vorrichtung nach einem der vorhergehenden Ansprüche, ***dadurch gekennzeichnet, daß*** der Bluteinlauf (14) einen Zyklon (13) aufweist.

7. Vorrichtung nach einem der vorhergehenden Ansprüche, ***dadurch gekennzeichnet, daß*** der Zyklon (13) zumindest teilweise in einem Rohr (2) angeordnet ist.

8. Vorrichtung nach einem der vorhergehenden Ansprüche, ***dadurch gekennzeichnet, daß*** der Blutauslauf (18) als Ringkanal mit in Strömungsrichtung zunehmendem Querschnitt ausgebildet ist.

9. Vorrichtung nach einem der vorhergehenden Ansprüche, ***dadurch gekennzeichnet, daß*** in einer Kammer (6) poröse Hohlkammermembranen (8) angeordnet sind.

## Claims

1. A device for the treatment of liquids, especially of blood, with several chambers (5, 6) formed by tubes (2, 3, 4) arranged inside one another, having an inlet (14) and an outlet (18) and in which hollow chamber membranes (8, 9) are arranged, while the chambers (5, 6) are so interconnected and inlet (14) and outlet (18) are so arranged that a flow in longitudinal direction of the tubes (2, 3, 4) is created in the chambers (5, 6), characterized by
- various types of hollow chamber membranes (8, 9) arranged in the individual chambers (5, 6),
- an upper and a lower lid (19, 20) with connections (22, 27, 31, 32) and
- webs (19) axially extending from the upper lid (19) to the tubes (2, 3), so that different media flow through the hollow chamber membranes (8, 9).

2. A device according to claim 1, characterized in that the tube (3) of the outer wall of an inner chamber (5) forms the inner wall of an outer chamber (6).

3. A device according to one of the preceding claims, characterized in that the chambers (5, 6) are largely of identical length and the axial ends of the hollow chamber membranes are situated in one plane.

4. A device according to one of the preceding claims, characterized in that the hollow chamber membranes (8, 9) are largely identical in length.

5. A device according to one of the preceding claims, characterized in that the tubes ((2, 3, 4) are designed with a slight taper.

6. A device according to one of the preceding claims, characterized in that the blood inlet (14) has a cyclone (13).

7. A device according to one of the preceding claims, characterized in that the cyclone (13), at least partly, is arranged in a tube (2).

8. A device according to one of the preceding claims, characterized in that the blood outlet (18) is designed as annular gallery with a cross section increasing with the direction of flow.

9. A device according to one of the preceding claims, characterized in that porous hollow chamber membranes (8) are arranged in a chamber (6).

## Revendications

1. Appareil de traitement de liquides, en particulier de sang, comprenant une multiplicité de chambres (5, 6) qui sont constituées de tubes (2, 3, 4) assemblés les uns dans les autres, et qui présentent une entrée (14) et une sortie (18), et dans lesquelles sont agencées des membranes alvéolaires (8, 9), lesdites chambres (5, 6) étant reliées l'une à l'autre et les entrée (14) et sortie (18) étant agencées de façon à générer, dans les chambres (5, 6), un écoulement dans le sens de la longueur des tubes (2, 3, 4), caractérisé par :
- différents types de membranes alvéolaires (8, 9) mises en place dans les chambres (5, 6),
- un couvercle supérieur et inférieur (19, 20) avec des raccords (22, 27, 31, 32) et
- des nervures s'étendant axialement, depuis le couvercle supérieur (19), vers les tubes (2, 3), de sorte que les membranes alvéolaires (8, 9) sont parcourues par des fluides différents.

2. Dispositif suivant la revendication 1, caractérisé en ce que le tube (3) de la paroi extérieure d'une chambre interne (5) forme la paroi intérieure d'une chambre externe (6).

3. Dispositif suivant l'une des revendications qui précèdent, caractérisé en ce que les chambres (5, 6) sont substantiellement de même longueur et en ce que les extrémités axiales des membranes alvéolaires se situent dans un plan.

4. Dispositif suivant l'une des revendications qui précèdent, caractérisé en ce que les membranes alvéolaires (8, 9) sont substantiellement de même longueur.

5. Dispositif suivant l'une des revendications qui précèdent, caractérisé en ce que les tubes (2, 3, 4) adoptent une forme légèrement conique.

6. Dispositif suivant l'une des revendications qui précèdent, caractérisé en ce que l'entrée (14) pour le sang comporte un cyclone (13).

7. Dispositif suivant l'une des revendications qui précèdent, caractérisé en ce que le cyclone (13) est assemblé, au moins en partie, dans un tube (2).

8. Dispositif suivant l'une des revendications qui précèdent, caractérisé en ce que la sortie (18) pour le sang est réalisée sous forme de passage annulaire de section transversale croissante dans le sens de l'écoulement.

9. Dispositif suivant l'une des revendications qui précèdent, caractérisé en ce que des membranes alvéolaires poreuses (8) sont logées dans une chambre (6).
